# FASCICULE DE BREVET EUROPEEN

(11) **EP 1 543 843 B1**
(45) Date de publication et mention de la délivrance du brevet: **14.02.2007**
(21) Numéro de dépôt: 04292870.5
(22) Date de dépôt: 03.12.2004
(51) Int. Cl.: A61L 9/12

(54) **Dispositif d'aération muni d'un diffuseur de parfum couplé à la commande d'obturation de l'aération**
Belüftungsvorrichtung mit einem an die Absperreinrichtung der Belüftung gekoppelten Duftspender
Aeration device with a fragrance dispenser coupled to the shutting device of the aeration

(30) Priorité: 19.12.2003 FR 0315074
(43) Date de publication de la demande: 22.06.2005
(73) Titulaire: Peugeot Citroën Automobiles S.A., 78140 Vélizy Villacoublay (FR)
(72) Inventeur: Dufeil. Laurent, 49240 Avrille (FR); George, Aubert, 78750 Mareil-Marly (FR); Guerin, Fabien, 92320 Chatillon (FR)
(74) Mandataire: Thinat, Michel

(56) Documents cités:
- DE-C- 19 529 451
- FR-A- 2 752 775
- GB-A- 2 322 554
- US-A- 4 677 902

## Description

L'invention concerne en général les dispositifs d'aération d'habitacles, et notamment d'habitacles de véhicules automobiles comme divulgués dans le document US 4677902.

Plus précisément, l'invention concerne selon un premier aspect un dispositif d'aération de l'habitacle d'un véhicule, notamment d'un véhicule automobile, comprenant un conduit d'aération débouchant à l'intérieur de l'habitacle, un organe d'obturation adoptant sélectivement une position d'obturation du conduit d'aération ou une position de dégagement de ce conduit, et un dispositif de commande de l'organe d'obturation.

Des dispositifs de ce type sont connus de l'art antérieur, et équipent pratiquement tous les véhicules automobiles actuels. Le conduit d'aération débouche dans l'habitacle au travers d'ouïes, l'organe d'obturation étant constitué par un volet situé en amont dans le conduit ou par les ouïes elles-mêmes.

Par ailleurs, il est courant de disposer un diffuseur de parfum dans l'habitacle du véhicule. Ce diffuseur est typiquement constitué d'un flacon rempli d'une substance parfumée sous forme solide, par exemple des granulés poreux imbibés d'un parfum liquide. Ces diffuseurs présentent le défaut que le parfum s'évente très vite si on laisse le flacon continuellement ouvert dans l'habitacle. Le conducteur doit donc boucher le flacon à chaque fois qu'il quitte le véhicule et le déboucher quand il remonte à bord de celui-ci.

Dans ce contexte, la présente invention a pour but de proposer un dispositif d'aération intégrant un diffuseur de parfum qui permette de pallier le défaut mentionné ci-dessus de façon simple, tout en profitant du courant d'air de ventilation pour diffuser le parfum.

A cette fin, le dispositif de l'invention, par ailleurs conforme à la définition générique qu'en donne le préambule ci-dessus, est essentiellement caractérisé en ce qu'il comprend un diffuseur de parfum et un organe de fermeture adoptant sélectivement une position de fermeture dans laquelle le diffuseur est isolé du conduit ou une position d'ouverture dans laquelle le diffuseur communique avec le conduit , et en ce que le dispositif de commande de l'organe d'obturation commande également l'organe de fermeture.

Dans un mode de réalisation possible de l'invention, le dispositif de commande adopte sélectivement des configurations initiale, intermédiaire et finale, l'organe d'obturation étant en position d'obturation et l'organe de fermeture étant en position de fermeture dans ladite configuration initiale, l'organe d'obturation étant en position de dégagement et l'organe de fermeture étant en position de fermeture dans ladite configuration intermédiaire, et enfin l'organe d'obturation étant en position de dégagement et l'organe de fermeture étant en position d'ouverture dans ladite configuration finale.

Avantageusement, l'organe de fermeture est un tiroir mobile longitudinalement relativement au conduit entre ses positions d'ouverture et de fermeture.

De préférence, le dispositif de commande comprend une molette de commande mobile en rotation autour d'un premier axe transversal entre des positions angulaires initiale, intermédiaire et finale correspondant respectivement aux configurations initiale, intermédiaire et finale du dispositif de commande, et un bras pivotant lié d'un côté par une liaison pivot autour d'un axe transversal au tiroir, et monté d'un côté opposé par une liaison pivot autour d'un autre axe transversal sur la molette, le déplacement de la molette de sa position angulaire initiale à sa position intermédiaire ou à sa position finale entraînant le tiroir de sa position de fermeture respectivement à une position longitudinale intermédiaire dans laquelle le diffuseur est isolé du conduit, ou à sa position d'ouverture.

Par exemple, l'organe d'obturation est un volet mobile en rotation autour d'un second axe transversal relativement au conduit entre ses positions d'obturation et de dégagement.

Suivant une autre caractéristique avantageuse de l'invention, la molette passe de sa position angulaire initiale à sa position finale par l'intermédiaire de sa position intermédiaire, par une rotation continue dans un même premier sens.

Dans ce cas, le dispositif de commande comprend un bras de commande radial solidaire de la molette, et une plaque solidaire du volet en rotation autour du second axe et s'étendant dans un plan sensiblement perpendiculaire à la direction transversale , la plaque comprenant une lumière dans laquelle est engagé un ergot porté par le bras de commande, le déplacement de la molette de sa position angulaire initiale à sa position intermédiaire provoquant le basculement du volet de sa position d'obturation à sa position d'ouverture sous l'effet du déplacement de l'ergot le long d'une première branche de la lumière.

En outre, quand la molette passe de sa position angulaire intermédiaire à sa position finale, l'ergot se déplace le long d'une seconde branche de la lumière formant un arc de cercle autour du premier axe transversal, de telle sorte que la plaque ne se déplace pas.

Alternativement, le dispositif de commande peut comprendre un levier solidaire du volet en rotation autour du second axe transversal, et un bras articulé constitué de premier et second segments mutuellement articulés, le premier segment étant lié d'un côté opposé au second segment par une liaison pivot d'axe transversal au levier, et le second segment étant solidaire de la molette, les premier et second segments étant mutuellement alignés dans la position angulaire initiale de la molette, et formant mutuellement un angle non nul l'un avec l'autre dans les positions angulaires intermédiaire et finale de la molette.

Dans ce cas, la molette passe de sa position angulaire initiale à sa position angulaire intermédiaire par rotation dans un premier sens à partir de la position initiale, ce qui entraîne la flexion du bras articulé d'un premier côté, la molette passant de sa position angulaire initiale à sa position angulaire finale par rotation dans un second sens opposé au premier à partir de la position initiale, ce qui entraîne la flexion du bras articulé d'un second côté opposé au premier.

Selon un second aspect, l'invention concerne le dispositif de commande des organes d'obturation et de fermeture du dispositif d'aération présentant les caractéristiques décrites ci-dessus.

D'autres caractéristiques et avantages de l'invention ressortiront clairement de la description qui en est faite ci-dessous, à titre indicatif et nullement limitatif, en référence aux figures annexées, parmi lesquelles :
- la figure 1 est une vue en perspective d'une partie de la planche de bord d'un véhicule équipé d'un dispositif d'aération selon l'invention, montrant la molette de commande,
- les figures 2A à 2C sont des vues en perspective d'un premier mode de réalisation du dispositif de commande du dispositif d'aération de l'invention, respectivement en configuration initiale, intermédiaire et finale,
- la figure 3 est une vue suivant la direction transversale du dispositif de commande des figures 2A à 2C, représenté en traits forts en position finale, en traits mixtes trait/trois points/trait en position intermédiaire, et en traits mixtes trait/point/trait en position initiale, et
- les figures 4A à 4C sont des vues équivalentes à celles des figures 2A à 2C, pour un second mode de réalisation de l'invention.

L'invention concerne un dispositif d'aération de l'habitacle d'un véhicule, notamment d'un véhicule automobile, comprenant un conduit d'aération 1 débouchant à l'intérieur de l'habitacle, un organe d'obturation 10 adoptant sélectivement une position d'obturation du conduit d'aération 1 ou une position de dégagement de ce conduit 1, et un dispositif 20 de commande de l'organe d'obturation 10.

Un flux d'air venant de l'extérieur traverse le conduit d'aération 1 et pénètre dans l'habitacle, typiquement à travers une ouïe, quand l'organe d'obturation 10 est en position de dégagement.

Le dispositif de commande 20 est actionnable manuellement à partir de l'intérieur de l'habitacle, les passagers pouvant ainsi choisir de ventiler ou non l'habitacle par l'intermédiaire de ce conduit 1.

Selon l'invention, le dispositif d'aération comprend un diffuseur de parfum 40, et un organe de fermeture 50 adoptant sélectivement une position de fermeture dans laquelle le diffuseur 40 est isolé du conduit 1, ou une position d'ouverture dans laquelle le diffuseur 40 communique avec le conduit 1, le dispositif de commande 20 de l'organe d'obturation 10 commandant également l'organe de fermeture 50.

Le dispositif de commande 20 adopte sélectivement des configurations initiale, intermédiaire et finale, au choix du passager du véhicule.

Dans la configuration initiale, l'organe d'obturation 10 est en position d'obturation et l'organe de fermeture 50 est en position de fermeture. L'air extérieur ne pénètre pas dans l'habitacle à travers le conduit 1, et le diffuseur de parfum 40 est isolé, de façon à ne pas s'éventer. Cette configuration initiale est typiquement choisie quand le véhicule est à l'arrêt et qu'aucun passager n'est à bord.

Dans la configuration intermédiaire, l'organe d'obturation 10 est en position de dégagement et l'organe de fermeture 50 est en position de fermeture. L'habitacle est ventilé à travers le conduit 1, mais le diffuseur de parfum reste isolé. Cette configuration est utilisée quand les passagers veulent une simple ventilation de l'habitacle, par un air non parfumé.

Enfin, dans la configuration finale, l'organe d'obturation 10 est en position de dégagement et l'organe de fermeture 50 est en position d'ouverture. Le parfum se diffuse à partir du diffuseur de parfum 40 dans le conduit 1, et est entraîné jusque dans l'habitacle par l'air venant de l'extérieur et traversant le conduit. Cette configuration est choisie quand les passagers veulent parfumer l'habitacle.

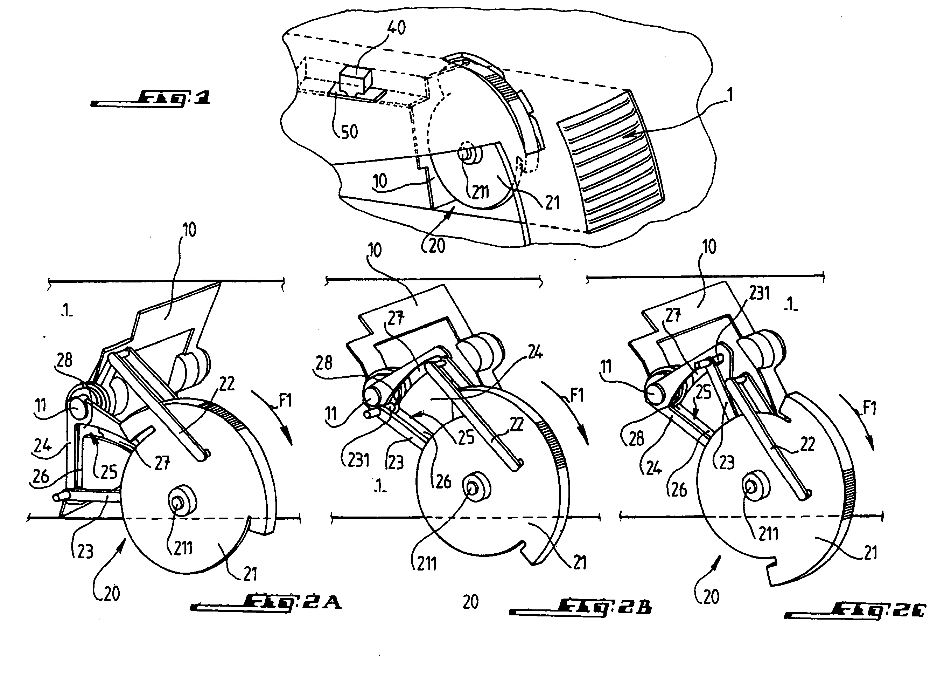

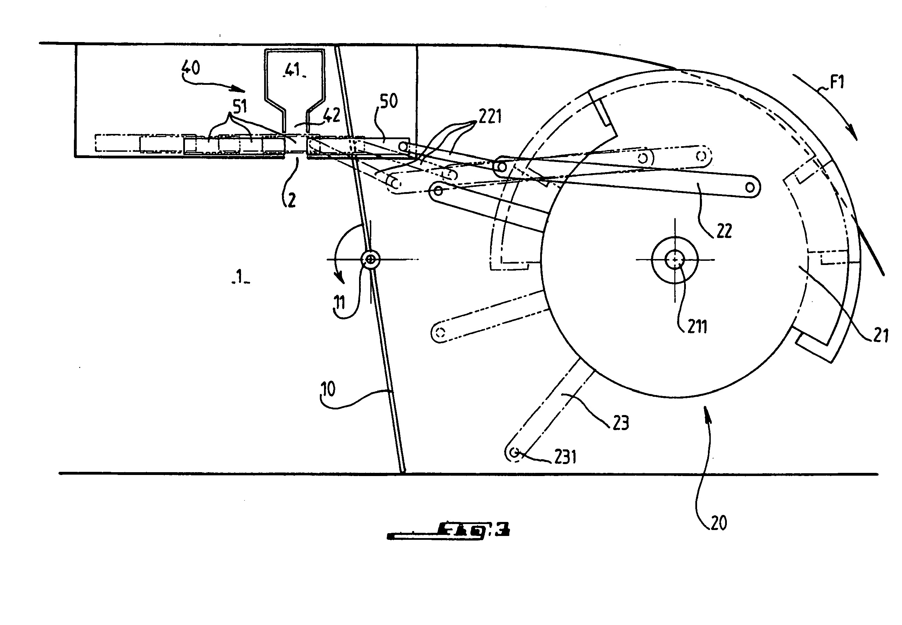

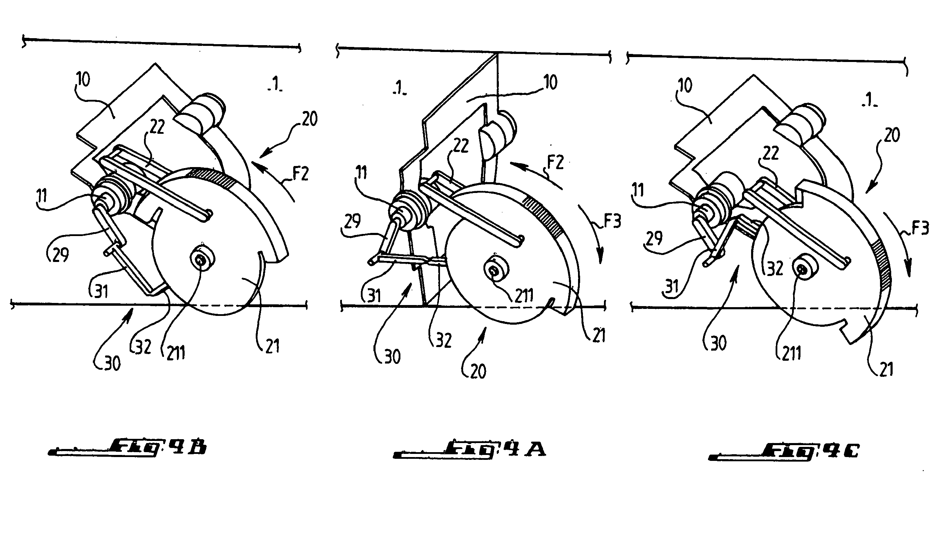

Comme le montre la figure 3, le diffuseur de parfum 40 est typiquement un flacon comprenant un volume intérieur 41 et un goulot 42 mettant en communication le volume intérieur 41 et le conduit 1. Le volume intérieur 41 ne communique avec l'extérieur du flacon que par le goulot 42.

Des granulés parfumés sont disposés dans le volume intérieur 41, ces granulés étant par exemple constitués d'une matière poreuse imbibée de liquide parfumé.

Le flacon 40 est disposé généralement à l'extérieur du conduit 1, de telle sorte que le goulot 42 soit situé en regard d'un orifice 2 ménagé dans la paroi du conduit 1. Comme le montre la figure 3, le flacon 40 peut être disposé au-dessus du conduit 1, l'ouverture du goulot étant tournée vers le bas. Une grille, non représentée, est alors disposée dans l'espace intérieur 41 pour retenir les granulés et les empêcher de tomber à l'intérieur du conduit 1.

L'organe de fermeture 50 est un tiroir mobile longitudinalement relativement au conduit 1 entre ses positions d'ouverture, de fermeture et une position longitudinale intermédiaire.

Le tiroir se déplace le long d'une face extérieure du conduit 1, et est interposé entre le goulot 42 et le conduit 1.

Ce tiroir 50 présente une ouverture 51, qui vient se positionner entre le goulot 42 et l'orifice 2 du conduit en position d'ouverture du tiroir 50, créant ainsi la communication entre l'espace intérieur 41 du flacon et l'intérieur du conduit. Dans ses positions de fermeture et intermédiaire, ce sont des parties pleines du tiroir 50 qui sont interposées entre le goulot 42 et l'orifice 2, isolant ainsi l'espace intérieur 41 de l'intérieur du conduit 1.

Le dispositif de commande 20 comprend une molette de commande 21 mobile en rotation autour d'un premier axe transversal 211 entre des positions angulaires initiale, intermédiaire et finale.

Comme le montre la figure 1, la molette 21 affleure sur la planche de bord du véhicule et peut être déplacée en rotation manuellement par un passager.

La molette 21 commande le passage du dispositif de commande 20 d'une configuration à une autre. Ainsi, les positions angulaires initiale, intermédiaire et finale de la molette 21 correspondent respectivement aux configurations initiale, intermédiaire et finale du dispositif de commande 20, le passage de la molette 21 d'une première position angulaire à une seconde provoquant le passage du dispositif de commande 20 de la configuration correspondant à la première position angulaire à la configuration correspondant à la seconde.

Le dispositif de commande 20 comprend encore un bras pivotant 22 lié d'un côté par une liaison pivot autour d'un axe transversal au tiroir 50, et monté d'un côté opposé par une liaison pivot autour d'un autre axe transversal sur la molette 21.

Le bras pivotant 22 est lié à une extrémité longitudinale du tiroir 50 tourné vers la molette 21 par l'intermédiaire d'une biellette 221. La biellette 221 est articulée à ses deux extrémités opposées, par des liaisons pivots d'axes transversaux, sur le tiroir 50 et sur une extrémité du bras pivotant 22.

Ce bras est lié à la molette 21 en un point d'articulation situé radialement environ à mi-distance entre le premier axe transversal 211 et un bord radialement extérieur de la molette 21.

Ainsi, le déplacement de la molette 21 de sa position angulaire initiale à sa position intermédiaire ou à sa position finale entraîne le tiroir 50 en translation longitudinale de sa position de fermeture respectivement à une position longitudinale intermédiaire ou à sa position d'ouverture.

On notera que le point d'articulation du bras pivotant 22, considéré suivant une direction transversale comme sur la figure 3, reste sensiblement dans le prolongement du tiroir 50 quand la molette 21 passe de sa position angulaire initiale à sa position finale. Cette disposition permet de transformer, de façon efficace et simple, le mouvement de rotation du point d'articulation en un mouvement de translation du tiroir. On notera en revanche que le bras articulé 22 et le tiroir 50 peuvent être décalés latéralement.

On notera encore que le conduit 1 porte des moyens de guidage en translation du tiroir 50, non représentés.

L'organe d'obturation 10 est un volet disposé dans le conduit 1 et mobile en rotation autour d'un second axe transversal 11 relativement au conduit 1 entre ses positions d'obturation et de dégagement.

Dans un premier mode de réalisation de l'invention représenté sur les figures 2A à 2C, la molette 21 se déplace de sa position angulaire initiale à sa position finale en passant par sa position intermédiaire, ce mouvement correspondant à une rotation continue de la molette 21 dans un même premier sens, représenté par la flèche F1 sur les figures 2A à 2C. La position angulaire intermédiaire est donc située entre les positions initiale et finale.

Dans ce cas, le dispositif de commande comprend un bras de commande 23 radial solidaire de la molette 21, et une plaque 24 solidaire du volet 21 en rotation autour du second axe 11 et s'étendant dans un plan sensiblement perpendiculaire à la direction transversale, la plaque 24 comprenant une lumière 25 dans laquelle est engagé un ergot 231 porté par une extrémité libre du bras de commande 23.

Le bras de commande 23 fait saillie radialement à partir d'un bord radialement extérieur de la molette 21, du côté du volet 10. L'ergot 231 s'étend transversalement à partir d'une extrémité libre du bras 23 opposée à la molette 21. Le bras est rectiligne.

La plaque 24 présente une forme sensiblement triangulaire, le second axe transversal 11 traversant la plaque à un sommet de celle-ci.

Comme on le voit sur la figure 2A, la lumière 25 présente une forme générale en V et comprend une première branche 26 rectiligne et une seconde branche 27 en arc de cercle, ces branches se rejoignant en un point de jonction 28 à proximité du second axe transversal 11.

En configuration initiale du dispositif de commande 20, l'ergot 231 occupe une extrémité de la première branche opposée au point de jonction 28. La première branche 26 s'étend alors dans une direction sensiblement perpendiculaire au bras de commande 23, la seconde branche 27 s'étendant à partir du point de jonction 28 sensiblement radialement vers le premier axe transversal 211.

Comme le montre la figure 2B, le déplacement de la molette 21 de sa position angulaire initiale à sa position intermédiaire provoque la rotation de la plaque 24 autour du second axe transversal 11 sous l'effet du déplacement de l'ergot 231 le long de la première branche 26. La rotation de la plaque entraîne le basculement du volet 10 de sa position d'obturation à sa position d'ouverture.

En effet, la molette 21 entraîne en rotation le bras de commande 23, ce qui provoque le déplacement de l'ergot 231 jusqu'au point de jonction 28. Pendant ce déplacement, l'ergot 231 sollicite un bord de la première branche 26 initialement tourné vers la molette 21 et provoque ainsi la rotation de la plaque 24.

Dans la configuration intermédiaire du dispositif de commande 20, la première branche 26 s'étend à partir du point de jonction 28 vers le premier axe 211, suivant une direction légèrement inclinée par rapport à la direction radiale. La seconde branche 27 forme alors un arc de cercle centré sur le premier axe 211.

Quand la molette 21 passe de sa position angulaire intermédiaire à sa position finale, l'ergot se déplace alors le long de la seconde branche 27 de la lumière 25, sans solliciter la plaque 24, de telle sorte que celle-ci ne se déplace pas. Le volet 10 reste donc en position de dégagement pendant ce mouvement.

Quand la molette 21 passe de sa position angulaire finale à sa position angulaire initiale, la plaque 24 et le bras de commande 23 effectuent des mouvements exactement inverses de ce qui a été décrit.

On notera que, dans ce premier mode de réalisation, une extrémité du tiroir 50 est située en regard du goulot 42 en configuration initiale du dispositif de commande 20. L'ouverture 51 est située dans une partie centrale du tiroir 50. Dans l'exemple de réalisation représenté sur les figures 2A à 2C et 3, l'extrémité du tiroir 50 située en regard du goulot 42 en configuration initiale est celle qui est liée du bras pivotant 22.

Dans un second mode de réalisation de l'invention représenté sur les figures 4A à 4C, la molette passe de sa position angulaire de départ à sa position angulaire intermédiaire par rotation dans un premier sens à partir de la position de départ, et la molette passe de sa position angulaire de départ à sa position angulaire finale par rotation dans un second sens opposé au premier à partir de la position de départ. Ces mouvements sont représentés par les flèches F2 et F3 des figures 4B et 4C.

Dans ce cas, le dispositif de commande 20 comprend un levier 29 solidaire du volet 10 en rotation autour du second axe transversal 11, et un bras articulé 30 constitué de premier et second segments 31 et 32 mutuellement articulés.

Le levier 29 est solidaire du volet 10 par une extrémité disposée dans le prolongement du second axe 11 et est lié au premier segment 31 par une extrémité opposée. Il s'étend dans un plan perpendiculaire à la direction transversale.

Le premier segment 31 est rectiligne. Il est lié par une extrémité au second segment 32 et est lié par une autre extrémité opposée au second segment 32 au levier 29 par l'intermédiaire d'une liaison pivot d'axe transversal.

Le second segment 32 est solidaire de la molette 21 et fait saillie radialement à partir d'un bord radialement extérieur de celle-ci du côté du volet 10. Ce second segment 32 est rectiligne et est lié au premier segment 31 par une extrémité opposée à la molette 21.

L'articulation entre les premier et second segments 31 et 32 est constituée d'une zone venue de matière avec ces deux segments, particulièrement fine, de telle sorte que les deux segments peuvent pivoter l'un par rapport à l'autre.

Les premier et second segments 31 et 32 sont mutuellement alignés dans la position angulaire initiale de la molette 21, représentée sur la figure 4A, suivant une direction radiale par rapport au premier axe 211. Ils forment mutuellement un angle non nul l'un avec l'autre dans les positions angulaires intermédiaire et finale de la molette 21, représentées sur les figures 4B et 4C.

Plus précisément, quand la molette 21 passe de sa position angulaire initiale à sa position angulaire intermédiaire par rotation dans le premier sens, le second segment 32 subit une rotation autour du premier axe 211, ce qui déplace l'articulation du bras d'un premier côté et entraîne la flexion de ce bras articulé 30 de ce premier côté. L'articulation se déplace dans un plan perpendiculaire à la direction transversale, suivant une direction perpendiculaire à la position initiale du bras articulé 30.

Sous l'effet de cette flexion, l'extrémité du premier segment 31 liée au levier 29 se rapproche du premier axe 211 en se déplaçant suivant une direction sensiblement radiale par rapport à celui-ci. Ce déplacement fait pivoter le levier 29 autour du second axe 11, ce qui entraîne le volet 10 en rotation de sa position d'obturation à sa position de dégagement.

Parallèlement, le tiroir 50 se déplace longitudinalement dans une première direction jusqu'à sa position longitudinale intermédiaire.

Quand la molette 21 passant de sa position angulaire initiale à sa position angulaire finale par rotation dans le second sens opposé au premier, le second segment 32 subit une rotation autour du premier axe 211, ce qui déplace l'articulation du bras d'un second côté et entraîne la flexion de ce bras articulé 30 de ce second côté. L'articulation se déplace dans un plan perpendiculaire à la direction transversale, suivant une direction perpendiculaire à la position initiale du bras articulé 30.

De même que précédemment, l'extrémité du premier segment 31 liée au levier 29 se rapproche du premier axe 211 sous l'effet de cette flexion, en se déplaçant suivant une direction sensiblement radiale par rapport à celui-ci. Ce déplacement fait pivoter le levier 29 autour du second axe 11, ce qui entraîne le volet 10 en rotation de sa position d'obturation à sa position de dégagement.

Parallèlement, le tiroir 50 se déplace longitudinalement dans une seconde direction opposée à la première jusqu'à sa position finale.

On comprend donc bien que dans ce second mode de réalisation de l'invention, une partie centrale du tiroir 50 est disposée en regard du goulot 42 du diffuseur de parfum en configuration de fermeture du dispositif de commande 20. L'ouverture 51 est située d'un côté longitudinal de cette partie centrale. Dans l'exemple de réalisation des figures 4A à 4C, le bras pivotant 22 est lié d'un côté longitudinal du tiroir 50 opposé à l'ouverture 51.

On notera que le dispositif d'aération décrit ci-dessus peut présenter de multiples variantes sans sortir du cadre de l'invention.

Le diffuseur de parfum 40 peut être disposé à l'intérieur du conduit 1, le tiroir 50 étant alors monté coulissant le long d'une face intérieure de ce conduit 1.

Dans le premier mode de réalisation de l'invention, la molette peut passer de sa position angulaire initiale à sa position angulaire finale par rotation dans un premier sens, par exemple vers le bas, ou dans le sens opposé, vers le haut. La position de l'orifice du tiroir doit être adaptée à chaque cas.

Le diffuseur de parfum peut contenir, à la place de la matière poreuse imbibée de parfum liquide, un liquide parfumé peu volatil, un gel parfumé, une matière solide parfumée, par exemple une matière organique.

L'invention n'est pas non plus limitée par la forme de la lumière décrite ci-dessus. La première branche 26 n'est pas nécessairement droite. Elle peut prendre par exemple une forme incurvée, déterminée de façon à demander un effort plus ou moins grand de l'utilisateur pour faire pivoter la molette. De même, le point de jonction 28 n'est pas nécessairement à proximité immédiate du second axe transversal 11, de façon à conserver un bras de levier important quand l'ergot 213 approche du point de jonction 28.

L'articulation des premier et second segments du bras articulé 30 peut être réalisée différemment de ce qui a été décrit ci-dessus, par exemple par une liaison pivot du type charnière.

La molette peut être remplacée par un levier de commande mobile en rotation autour du premier axe transversal pour passer de la position angulaire initiale aux positions intermédiaire et finale.

Le dispositif d'aération peut être utilisé sur tous les véhicules comprenant des habitacles fermés, et peut être adapté à un véhicule automobile, à un wagon de chemin de fer, en particulier aux wagons comportant des compartiments séparés, ou même à un avion.

Dans le cas d'un véhicule automobile, le diffuseur de parfum peut être installé dans le conduit menant à une ouïe latérale, à une ouïe centrale, ou encore à une fente de ventilation du pare-brise.

On comprend bien que le dispositif de l'invention présente des avantages considérables.

Il offre trois configurations sélectionnables au choix de l'utilisateur: pas de ventilation, ventilation de l'habitacle sans parfum, ventilation de l'habitacle avec parfum.

Un dispositif de commande unique permet de basculer simplement et rapidement d'une configuration à une autre.

Ce dispositif de commande est très discret, puisque la seule modification apparente pour l'utilisateur par rapport à l'art antérieur est de rajouter une position supplémentaire pour la molette de commande du dispositif d'aération.

Isoler le diffuseur de parfum ne demande pas d'effort particulier à l'utilisateur. Il n'a pas à rechercher un bouchon et ne risque pas de perdre celui-ci. Il exécute cette opération d'un même mouvement qu'il obture le conduit d'aération.

Par ailleurs, la situation « normale » du dispositif d'aération est celle dans laquelle le conduit est obturé. Le dispositif de l'invention incite donc l'utilisateur à ne mettre en service le diffuseur de parfum que quand il le souhaite expressément. Ce n'était pas le cas dans l'art antérieur, la plupart des utilisateurs laissant le diffuseur de parfum ouvert en permanence, même sans raison particulière, ce qui conduit à éventer très rapidement et inutilement ce diffuseur.

Enfin, le dispositif permet de profiter de façon très efficace de l'air de ventilation pour diffuser le parfum, puisque ce parfum est relâché dans le conduit d'aération.

## Revendications

1. Dispositif d'aération de l'habitacle d'un véhicule, notamment d'un véhicule automobile, comprenant un conduit d'aération (1) débouchant à l'intérieur de l'habitacle, un organe d'obturation (10) adoptant sélectivement une position d'obturation du conduit d'aération (1) ou une position de dégagement de ce conduit, et un dispositif (20) de commande de l'organe d'obturation (10), un diffuseur de parfum (40), et un organe de fermeture (50) adoptant sélectivement une position de fermeture dans laquelle le diffuseur (40) est isolé du conduit (1) ou une position d'ouverture dans laquelle le diffuseur (40) communique avec le conduit (1) , **caractérisé en ce que** le dispositif de commande (20) de l'organe d'obturation (10) commande également l'organe de fermeture (50).

2. Dispositif selon la revendication 1, **caractérisé en ce que** le dispositif de commande (20) adopte sélectivement des configurations initiale, intermédiaire et finale, l'organe d'obturation (10) étant en position d'obturation et l'organe de fermeture (50) étant en position de fermeture dans ladite configuration initiale, l'organe d'obturation (10) étant en position de dégagement et l'organe de fermeture (50) étant en position de fermeture dans ladite configuration intermédiaire, et enfin l'organe d'obturation (10) étant en position de dégagement et l'organe de fermeture (50) étant en position d'ouverture dans ladite configuration finale.

3. Dispositif selon la revendication 2, **caractérisé en ce que** l'organe de fermeture (50) est un tiroir mobile longitudinalement relativement au conduit (1) entre ses positions d'ouverture et de fermeture.

4. Dispositif selon la revendication 3, **caractérisé en ce que** le dispositif de commande (20) comprend une molette de commande (21) mobile en rotation autour d'un premier axe transversal (211) entre des positions angulaires initiale, intermédiaire et finale correspondant respectivement aux configurations initiale, intermédiaire et finale du dispositif de commande (20), et un bras pivotant (22) lié d'un côté par une liaison pivot autour d'un axe transversal sur le tiroir (50), et monté d'un côté opposé par une liaison pivot autour d'un autre axe transversal sur la molette (21), le déplacement de la molette (21) de sa position angulaire initiale à sa position intermédiaire ou à sa position finale entraînant le tiroir (50) de sa position de fermeture respectivement à une position longitudinale intermédiaire dans laquelle le diffuseur (40) est isolé du conduit (1), ou à sa position d'ouverture.

5. Dispositif selon la revendication 4, **caractérisé en ce que** l'organe d'obturation (10) est un volet mobile en rotation autour d'un second axe transversal (11) relativement au conduit (1) entre ses positions d'obturation et de dégagement.

6. Dispositif selon la revendication 5, **caractérisé en ce que** la molette (21) passe de sa position angulaire initiale à sa position finale par l'intermédiaire de sa position intermédiaire, par une rotation continue dans un même premier sens.

7. Dispositif selon la revendication 6, **caractérisé en ce que** le dispositif de commande (20) comprend un bras de commande (23) radial solidaire de la molette (21), et une plaque (24) solidaire du volet (10) en rotation autour du second axe (11) et s'étendant dans un plan sensiblement perpendiculaire à la direction transversale, la plaque (24) comprenant une lumière (25) dans laquelle est engagé un ergot (231) porté par le bras de commande (23), le déplacement de la molette (21) de sa position angulaire initiale à sa position intermédiaire provoquant le basculement du volet (10) de sa position d'obturation à sa position d'ouverture sous l'effet du déplacement de l'ergot (231) le long d'une première branche (26) de la lumière (25).

8. Dispositif selon la revendication 7, **caractérisé en ce que**, quand la molette (21) passe de sa position angulaire intermédiaire à sa position finale, l'ergot (231) se déplace le long d'une seconde branche (27) de la lumière (25) formant un arc de cercle autour du premier axe transversal (211), de telle sorte que la plaque (24) ne se déplace pas.

9. Dispositif selon la revendication 5, **caractérisé en ce que** le dispositif de commande (20) comprend un levier (29) solidaire du volet (10) en rotation autour du second axe transversal (11), et un bras articulé (30) constitué de premier et second segments (31, 32) mutuellement articulés, le premier segment (31) étant lié d'un côté opposé au second segment (32) par une liaison pivot d'axe transversal au levier (29), et le second segment (32) étant solidaire de la molette (21), les premier et second segments (31, 32) étant mutuellement alignés dans la position angulaire initiale de la molette (21), et formant mutuellement un angle non nul l'un avec l'autre dans les positions angulaires intermédiaire et finale de la molette (21).

10. Dispositif selon la revendication 9, **caractérisé en ce que** la molette (21) passe de sa position angulaire initiale à sa position angulaire intermédiaire par rotation dans un premier sens à partir de la position initiale, ce qui entraîne la flexion du bras articulé (30) d'un premier côté, la molette (21) passant de sa position angulaire initiale à sa position angulaire finale par rotation dans un second sens opposé au premier à partir de la position initiale , ce qui entraîne la flexion du bras articulé (30) d'un second côté opposé au premier.

11. Dispositif de commande des organes d'obturation et de fermeture du dispositif d'aération selon l'une quelconque des revendications précédentes.

## Claims

1. Vehicle body aeration device, particularly one for motor vehicles, comprising an aeration conduit (1), opening into the inside of the body, a cover member (10) that selectively adopts a position covering the aeration conduit (1) or a position of clearance from this conduit, a device (20) for controlling the cover member (10), a perfume diffuser (40), and a closing body that selectively takes up a closing position in which the diffuser (40) is isolated from the conduit (1) or an open position in which the diffuser (40) communicates with the conduit (1), **characterized in that** the control device (20) of the covering body (10) also controls the closing member (50).

2. Device in accordance with claim 1, **characterized in that** the control device (20) selectively takes up initial, intermediate and final arrangements, the covering body (10) being in a covering position and the closing body (50) being in a closing position in the said initial configuration, the covering body (10) being in a clearance position and the closing body (50) being in a closing position in the said intermediate position and, finally, the cover body (10) being in a clearance position and the closing body (50) being in an opening position in the said final configuration.

3. Device in accordance with claim 2, **characterized in that** the closing body (50) is a sliding distributing regulator that can move longitudinally, relative to the conduit (1), between its opening and closing positions.

4. Device in accordance with claim 3, **characterized in that** the control device (20) comprises a control roller (21) that can move, in rotation around a first transverse axis (211), between the initial, intermediate and final angular positions corresponding to the initial, intermediate and final positions of the control device (20) respectively, and a swivel arm (22) connected on one side by a link pivoting around a transverse axis on the sliding distributing regulator (50) and, on an opposite side, mounted by a link pivoting around another transverse axis on the roller (21), the movement of the roller (21) from its initial, angular position to its intermediate position or to its final position drawing along the sliding distributing regulator (50) from its closing position to an intermediate, longitudinal position in which the diffuser (40) is isolated from the conduit (1), or to its opening position, respectively.

5. Device in accordance with claim 4, **characterized in that** the covering body (10) is a flap able to rotate, relative to the conduit (1), around a second transverse axis (11), between its positions of covering and of clearance.

6. Device in accordance with claim 5, **characterized in that** the roller (21) goes from its initial, angular position to its final position, passing through its intermediate position, by means of continuous rotation in one same, first direction.

7. Device in accordance with claim 6, **characterized in that** the control device (20) comprises a control arm (23) that, radially, functions in conjunction with the roller (21), and a plate (24) that functions in conjunction with the flap (10), rotating around the second axis (11) and extending in a plane that is markedly perpendicular to the transverse direction, the plate (24) comprising a light (25) in which a pin (231), carried by the control arm (23), is engaged, the movement of the roller (21), from its initial, angular position to its intermediate position, bringing about, under the effect of the movement of the pin (231) along a first branch (26) of the light (25), the tilting over of the flap (10) from its covering position to its opening position.

8. Device in accordance with claim 7, **characterized in that**, when the roller (21) passes from its intermediate, angular position to its final position, the pin (231) moves along the second branch (27) of the light (25), tracing a circular arc around the first transverse axis (211), such that the plate (24) does not move.

9. Device in accordance with claim 5, **characterized in that** the control device (20) comprises a lever (29), which functions in conjunction with the flap (10), that rotates around the second transverse axis (11), and an articulated bracket (30) made up of first and second segments (31, 32) that are mutually articulated, the first segment (31) being connected, on a side opposite to the second segment (32), by a swivel link of the transverse axis, to the lever (29) and the second segment (23) being joined to the roller (21), the first and second segments (31, 32) being mutually aligned in the initial angular position of the roller (21) and mutually forming a non-zero angle with the other in the intermediate and final positions of the roller (21).

10. Device in accordance with claim 9, **characterized in that** the roller (21) passes from its initial angular position to its intermediate, angular position, by rotating in a first direction from its initial position, this leading to turning of the articulated bracket (30) on one side, the roller (21) passing from its initial, angular position to its final, angular position by rotation in a second direction opposite to the first direction, from the initial position, leading to the turning of the articulated bracket (30) on a second side opposite the first.

11. Device for controlling covering and closing members of the aeration device in accordance with any one of the above claims.

## Patentansprüche

1. Vorrichtung zum Belüften des Fahrgastraums eines Fahrzeugs, insbesondere eines Kraftfahrzeugs, mit einer Belüftungsleitung (1), die in den Fahrgastraum mündet, einem Absperrorgan (10), das selektiv eine Stellung zum Absperren der Belüftungsleitung (1) bzw. eine Stellung zum Freigeben dieser Leitung einnimmt, und einer Einrichtung (20) zum Steuern des Absperrorgans (10), einem Duftspender (40) und mit einem Schließorgan (50), das selektiv eine Schließstellung einnimmt, in welcher der Duftspender (40) von der Leitung (1) getrennt ist, bzw. eine Öffnungsstellung, in welcher der Duftspender (40) mit der Leitung (1) kommuniziert, **dadurch gekennzeichnet, dass** die Einrichtung (20) zum Steuern des Absperrorgans (10) auch das Schließorgan (50) steuert.

2. Vorrichtung nach Anspruch 1, **dadurch gekennzeichnet, dass** die Steuereinrichtung (20) selektiv eine Ausgangs-, eine Zwischen- und eine Endgestalt annimmt, wobei in der Ausgangsgestalt das Absperrorgan (10) in Absperrstellung und das Schließorgan (50) in Schließstellung ist, in der Zwischengestalt das Absperrorgan (10) in Freigabestellung und das Schließorgan (50) in Schließstellung ist, und schließlich in Endgestalt das Absperrorgan (10) in Freigabestellung und das Schließorgan (50) in Öffnungsstellung ist.

3. Vorrichtung nach Anspruch 2, **dadurch gekennzeichnet, dass** das Schließorgan (50) ein relativ zur Leitung (1) zwischen ihrer Öffnungs- und Schließstellung longitudinal verstellbarer Schieber ist.

4. Vorrichtung nach Anspruch 3, **dadurch gekennzeichnet, dass** die Steuereinrichtung (20) ein Steuerrad (21) enthält, das zwischen einer Ausgangs-, einer Zwischen- und einer Endwinkelstellung um eine erste Querachse (211) drehbar ist, die der Ausgangs-, Zwischen- bzw. Endgestalt der Steuereinrichtung (20) entspricht, sowie einen Schwenkarm (22), der auf einer Seite über eine um eine Querachse verschwenkbare Verbindung an den Schieber (50) angefügt und auf der entgegengesetzten Seite über eine um eine weitere Querachse verschwenkbare Verbindung an das Steuerrad (21) montiert ist, wobei mit der Verstellung des Steuerrads (21) aus seiner Ausgangswinkelstellung in seine Zwischenstellung bzw. in seine Endstellung der Schieber (50) aus seiner Schließstellung in eine longitudinale Zwischenstellung, in welcher der Duftspender (40) von der Leitung (1) getrennt ist, bzw. in seine Öffnungsstellung mitgenommen wird.

5. Vorrichtung nach Anspruch 4, **dadurch gekennzeichnet, dass** das Absperrorgan (10) eine Klappe ist, die relativ zur Leitung (1) zwischen ihrer Absperrstellung und ihrer Freigabestellung um eine zweite Querachse (11) drehbar ist.

6. Vorrichtung nach Anspruch 5, **dadurch gekennzeichnet, dass** das Steuerrad (21) durch kontinuierliche Drehung in einer gleichen ersten Richtung aus seiner Ausgangswinkelstellung über seine Zwischenstellung in seine Endstellung gelangt.

7. Vorrichtung nach Anspruch 6, **dadurch gekennzeichnet, dass** die Steuereinrichtung (20) einen radialen Steuerarm (23) enthält, der fest mit dem Steuerrad (21) verbunden ist, sowie eine Platte (24), die um die zweite Achse (11) drehfest mit der Klappe (10) verbunden ist und sich in einer Ebene im wesentlichen senkrecht zur Querrichtung erstreckt, wobei die Platte (24) ein Langloch (256) aufweist, in welches ein Vorsprung (231) eingreift, der von dem Steuerarm (23) getragen wird, wobei die Verstellung des Steuerrads (21) aus seiner Ausgangswinkelstellung in seine Zwischenstellung das Verschwenken der Klappe (10) aus ihrer Absperrstellung in ihre Öffnungsstellung unter der Wirkung der Verstellung des Vorsprungs (231) entlang eines ersten Schenkels (26) des Langlochs (25) hervorruft.

8. Vorrichtung nach Anspruch 7, **dadurch gekennzeichnet, dass** dann, wenn das Steuerrad (21) aus seiner Zwischenwinkelstellung in seine Endstellung übergeht, der Vorsprung (231) sich entlang eines zweiten Schenkels (27) des Langlochs (25) verlagert, der einen Kreisbogen um die erste Querachse (211) bildet, so dass die Platte (24) sich nicht verstellt.

9. Vorrichtung nach Anspruch 5, **dadurch gekennzeichnet, dass** die Steuereinrichtung (20) einen Hebel (29) enthält, der um die zweite Querachse (11) drehfest mit der Klappe (10) verbunden ist, sowie einen Gelenkarm (30), der aus einem ersten und einem zweiten Segment (31, 32) besteht, die aneinander angelenkt sind, wobei das erste Segment (31) auf einer dem zweiten Segment (32) entgegengesetzten Seite über eine um die Querachse verschwenkbare Verbindung mit dem Hebel (29) verbunden ist, und das zweite Segment (32) fest mit dem Steuerrad (21) verbunden ist, wobei das erste und das zweite Segment (31, 32) in der Ausgangswinkelstellung des Steuerrads (21) miteinander fluchten und in der Zwischen- und der Endwinkelstellung des Steuerrads (21) einen Winkel ungleich null miteinander einschließen.

10. Vorrichtung nach Anspruch 9, **dadurch gekennzeichnet, dass** das Steuerrad (21) durch Drehung aus der Ausgangsstellung in einer ersten Richtung aus seiner Ausgangswinkelstellung in seine Zwischenwinkelstellung übergeht, wodurch die Biegung des Gelenkarms (30) auf einer ersten Seite hervorgerufen wird, wobei das Steuerrad (21) durch Drehung aus der Ausgangsstellung in einer der ersten Richtung entgegengesetzten zweiten Richtung aus seiner Ausgangswinkelstellung in seine Endwinkelstellung übergeht, wodurch die Biegung des Gelenkarms (30) auf einer der ersten Seite entgegengesetzten zweiten Seite hervorgerufen wird.

11. Einrichtung zum Steuern der Absperr- und Schließorgane der Belüftungsvorrichtung nach einem der vorangehenden Ansprüche.
